# EUROPEAN PATENT APPLICATION

(11) **EP 2 388 262 A1**
(43) Date of publication of application: **23.11.2011**
(21) Application number: 10005303.2
(22) Date of filing: 20.05.2010
(51) Int. Cl.: C07D 493/04

(54) **New biobased chiral compounds**

(71) Applicant: Stichting Dutch Polymer Institute, 5612 AB Eindhoven (NL)
(72) Inventor: The designation of the inventor has not yet been filed
(74) Representative: Renkema, Jaap

(57) **Abstract**

The present invention relates to a compound which has the general formula: 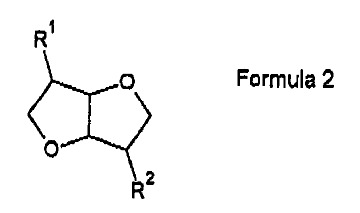 wherein the R¹ and R² may be the same or different and selected from -OH and a reactive group, the reactive group comprising a bridging carbon atom being directly bonded to the bicyclic isohexide, provided that R' and R² are not both -OH.

The present invention also relates to preparation methods for and uses of such compounds.

## Description

The present Invention relates to biobased chiral compounds derived from isohexides, The Invention also relates to the preparation and use of such compounds.

### BACKGROUND

In many studies on renewable resources, the easily accessible 1,4:3,6-dianhydrohexitols (Formula 1), also known as isohexides, have been Identified as so-called platform chemicals and have been attractive for industrial applications for several decades. When incorporated in polyesters such as PET, these rigid secondary diols impart high chain stiffness, resulting in an increased glass transition temperature (T_{g}).

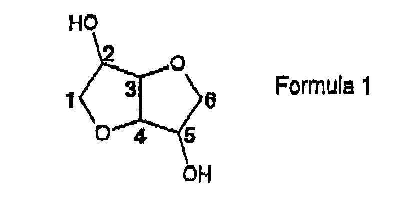

Due to the relatively low reactivity of isohexides in step-growth polymerization, there is still no commercial application In PET type polyesters with high molecular weights and high melting points. Until now, only a few types of low molecular weight or low melting point polyesters containing isohexides (mostly isosorbide) have been reported; e.g. poly isosorbide succinate, or low molecular weight polyesters for powder coating resins. Some other applications, like PEIT (poly(ethylene-co-isosorbide)terephthalate) developed by Dupont and Roquette, Involve using low percentages (10-20%) of isosorbide as a comonomer to increase the T_{g} above 100°C.

WO2008/145921 discloses compounds which are derived from isohexides and have the general formula: R*-(CH₂)₂-O-A-O-(CH₂)₂-R, wherein A is a divalent isohexide derived radical and -R is -CN or -CH₂NH₂.

US2008/0009599 discloses thermoset epoxy polymers using bisglycidyl ethers of anhydrosugars, such as isosorbide, isomannide and isoldide. It Is also disclosed that such bisglycidyl ethers are useful as substitutes for bisphenol A In the manufacture of thermoset epoxy ethers. It is further disclosed that it is desirable to use curing agents derived from renewable resources, which could for example be (3R,3aR,6S,6aR)-Hexahydro-furo[3,2-b]furan-3,6-diamine or Isosorbide 2,5-bis(3-aminopropyl)ether. The first having a formula similar to formula 1, wherein both OH-groups on the 2 and 5 position of the bicycllc ring have been substituted by -NH₂. The latter having a formula similar to formula 1, wherein both OH-groups have been substituted by -O-(CH₂)₃-NH₂.

A disadvantage of isohexides is that, when compared to e.g. ethylene glycol, isohexides are relatively unreactive secondary diols, requiring severe polymerization conditions (e.g. vacuum processing at reaction temperatures exceeding 180°C) and long polymerization times (e.g. over 6 hrs) in order to achieve the desired levels of incorporation. This in turn often leads to dark colors and unsatisfactory low molecular weights, in case of polymers for engineering plastics (see e.g. "Incorporation of Isosorbide into Poly(butylenes terephthalate) via Solid-State Polymerization", Rafael Sablong et al., Biomacromolecules, 2008, 9 (11), 3090-3097; and "Co- and Terpolyesters Based on Isosorbide and Succinic Acid for Coating Applications: Synthesis and Characterization", Bart A. J. Noordover et al. Biomacromoleculas 2006, 7, 3406-3416).

A disadvantage of the known derivatives of isohexides is that these compounds have a relatively long bridging group (i.e. -(CH₂)₂-O- in case of WO2008/146921 and -O-(CH₂)₃. In case of US2008/0009599) between the reactive groups (I.e. R' in case of WO2008/145921 and -NH_{z} In case of US2008/0009599) and the rigid parent isohexide (A in case of WO2008/145921). Compared to polymers prepared with isohexide monomers, polymers prepared with such derivatives do not have high rigidity in the polymer backbone, resulting in unsatisfactory low T_{g}'s.

A disadvantage of the known derivatives of isohexides is that the ether linkage In the bridging group (i.e. the -O- linkage) may be sensitive to actinic radiation, in particular UV-radiation. A product obtained from the known derivatives of isohexides as described above, e.g. polymers may therefore be sensitive to actinic radiation, In particular UV-radiation. Degradation of the product, in particular the polymer due to chain scission might occur under the Influence of actinic radiation, in particular UV-radiation. Such products and polymers might therefore also be sensitive to weathering. The degradation of the ether linkages in the bicyclic ring does not lead to chain scission and may leave the polymer backbone intact.

It is an object of the present invention to provide compounds obtained from renewable resources, the compounds being building blocks In all kinds of technologies, e.g. in the field of the manufacturing pharmaceuticals, In liquid crystal technology, as a ligand system for catalysis, and in polymer technology.

It is another object of the present invention that the biobased building blocks are chiral compounds, especially when used in pharmaceutical manufacturing, in liquid crystal technology and as ligand systems for catalysis.

It is yet another object of the present Invention that the compounds obtained from renewable resources show an increased reactivity, e.g. in polymerization processes, such that reaction (e.g. polymerization) under mild conditions Is possible, leading to shorter reaction times and improved product properties (e.g. improved color, high molecular weights and high T_{g}, in case of polymerization).

### SUMMARY OF THE INVENTION

This object Is achieved by providing a compound which has the general formula: 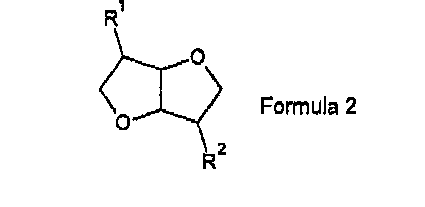 wherein the R¹ and R² may be the same or different and selected from -OH and a reactive group, the reactive group comprising a bridging carbon atom being directly bonded to the bicyclic isohexide, provided that R¹ and R² are not both -OH. Preferably R¹ and R² are Independently from one another selected from the group consisting of -OH; - CN; -COOH; -CH₂NH₂; -CH₂OH; -(C=O)-O-Rx; -CH₂NCO, and oxazoline, provided that R¹ and R² are not both -OH and wherein Rx may be a branched or unbranched C₁-C₂₂ alkyl-group, preferably a C₁-C₁₀ alkyl-group, more preferably a C₁-C₄ alkyl-group, more preferably an alkyl-group selected from the group consisting of methyl, ethyl, n-propyl, i-propyl, n-butyl, s-butyl, t-butyl. Most preferably Rx is methyl or ethyl. The alkyl-group Rx may be optionally substituted and optionally containing hetero-atoms such as S, O, N.

Such compounds may be derived from an isohexide and may show an improved (i.e. higher) reactivity and may allow reaction under mild processing conditions, such that products with improved properties may be obtained more easily. Such compounds are biobased chiral building blocks which may be of interest in the fields of:
- polymer technology
- manufacturing of pharmaceuticals;
- liquid crystal technology;
- ligand systems in catalysis.

The invention will now be explained In detail with reference to the following Figures.
- Figure 1: Schematic representation of a preparation route of a dinitrile derivative of an isohexide.
- Figure 2: Schematic representation of a procedure for preparing new isohexidesbased building blocks from the dinitrile derivative of an isohexide.
- Figure 3: Schematic representation of a preparation route of a mononitrile derivative of an isohexide.
- Figure 4: Schematic representation of a procedure for preparing new isohexidesbased building blocks from the mononitrile derivative of an isohexide.

### DETAILED DESCRIPTION

In an embodiment, R¹ and R² may be the same. The Isohexide derivatives according to this embodiment of the present invention comprise a new group of isohexide based compounds, which may be used as building blocks, e.g. in polymer technology, In the manufacturing of pharmaceuticals, In liquid crystal technology, or as ligand systems in catalysis. Such building blocks combine the rigidity of the parent isohexides with new, more reactive functional groups, which will allow for less severe reaction conditions.

For the purpose of the present invention the term isohexides will be used to Indicate all three stereo-isomers as shown below of 1,4:3,8-dianhydrohexitol.

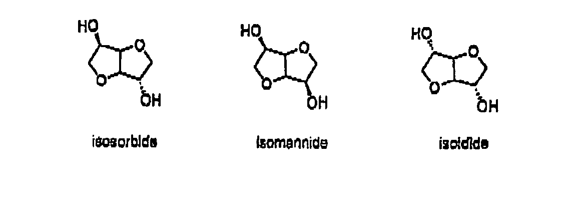

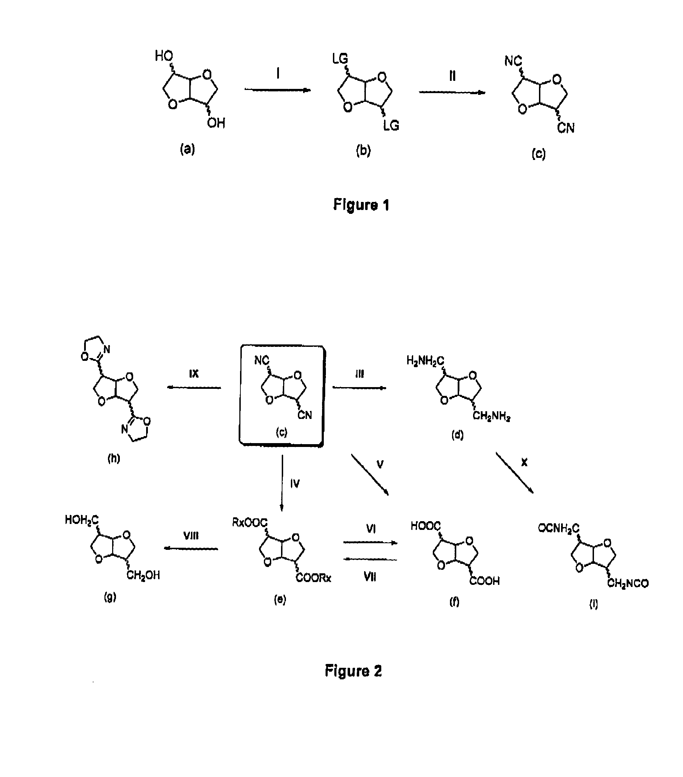

Based on the principle that, In order to retain the rigidity to obtain high T_{g}, the new reactive functionalities should be attached as close as possible to the bicyclic ring (3a,6a-dihydro-furo[3,2-b]furan), several groups of isohexide derivatives (e.g. diamines, diacids, diesters, diols, diisocyanates and bisoxazolines) were designed, as illustrated in Figure 2.

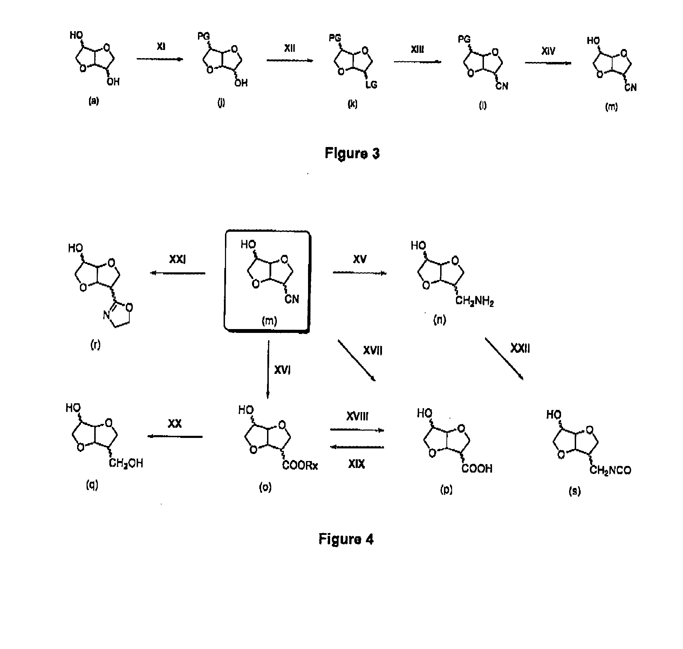

In an embodiment, compounds according to the present invention comprise mono-substituted derivatives of isohexides, as shown in Figure 4. The mono-substituted derivatives comprise aminoalcohol, hydroxyacids, monoesters, diols (comprising one - OH directly connected to the bicyclic isohexide and one -CH₂-OH), monoisocyanates and oxazolines. The compounds according to this embodiment have the general formula 2, wherein one of the R¹ and R² -groups comprises -OH and the other is selected from the group consisting of -CN, -COOH, -CH₂NH₂, -CH₂OH, -(C=O)-O-Rx, -CH₂NCO, and oxazoline; wherein Rx may be a branched or unbranched C₁∼C₂₂ alkyl-group, preferably a C₁-C₁₀ alkyl-group.

In an embodiment the isohexide from which the compound according to the present invention is derived is selected from the group consisting of isosorblde, isomannide and isoidide, preferably isomannide is selected. In substitution reactions, inversion of the isomannide conformation into the isoidide conformation takes place. The derivatives of isohexide having the isoldide conformation may show the highest reactivity. Due to the symmetry in the isoidide derivative, a product, In particular a polymer, obtained by reacting such derivatives, may show Increased crystallinity. On the other hand, from an availability and cost point of view, the isosorbide isomer may be preferred.

The potential commercial impact of compounds according to the present invention (and shown in e.g. Figure 2 and Figure 4) may be very high since isohexides are renewable, commercially available and can be obtained from existing renewable bulk feed stocks like starch, sorbitol, or cellulose.

Furthermore, all synthetic techniques used to obtain the new rigid building blocks may be easily implemented in common industrial practice. Given the fact that a whole new family of building blocks (diaclds, diester, diols, diamines, and corresponding diisocyanates, as well as bisoxazolines) can be prepared, which may be successfully used in various polymerization processes, even at low levels of Incorporation In copolymers (e.g. incorporation in PET).

The new diacid (Formula 2, wherein R¹ and R² are -COOH) could become a rigid bio-based, and thus renewable, alternative to terephthalic acid. This may suggest large scale potential In step-growth polymers (e.g. engineering plastics, coatings, etc.). Because of the obvious differences with petrochemical analogues (e.g. terephthalic acid) new polymeric materials with improved biodegradability, UV stability and/or moisture capacity can be envisaged.

Compounds according to the present Invention may therefore be used as bio-based or renewable building blocks in various polymerization reactions, for example for the preparation of renewable polyesters having a high T_{g}, or polyamldes which could exhibit lyotropic behavior in suitable solvents.

Furthermore, because of the chirality of the new molecules as shown in Figure 2 and In Figure 3, they could be applied e.g. in the manufacture of pharmaceuticals, in liquid crystal technology (e.g. as liquid crystalline materials, liquid crystalline polymers (LCP's), liquid crystalline coatings, display materials) and as ligand systems in catalysis.

### Process

A general process used for preparing the isohexide-based building blocks according to an embodiment of the present invention is depicted in Figures 1 and 2. A first and second step are shown in Figure 1. First (step I) an activated isohexide may be prepared by reacting an isohexide, represented by (a) In Figure 1, (i.e. isosorbide, isomannlde or isoidide) with a triflate reagent, e.g. (CF₃SO₂)₂O or CF₃SO₂Ci. The activated isohexide, (b), may have trlflate leaving groups (LG) introduced on the 2 and 5 position of the bicyclic isohexide (see also Formula 1). In a second step (step II), the activated isohexide may be reacted with e.g. NaCN in THF as a solvent In order to obtain a dinitrile derivative, (c), of the corresponding isohexide. The obtained dinitrile may be used as a platform chemical for preparing numerous derivatives of which 6 alternatives are shown in Figure 2.

Figure 2 shows that departing from the dinitrile derivative, (c), of the isohexide, a diamine, (d), may be obtained, by reacting the dinitrile with e.g. LiAlH₄ or H₂ and a catalyst (step 111), i.e. a reduction of the dinitrile into a diamine. Step IV shows the acid hydrolysis of the dinitrile into a diester, (e), by reacting the dinitrile with an alcohol with general formula Rx-OH, wherein Rx may be a branched or unbranched C₁-C₂₂ alkyl-group, preferably a C₁-C₁₀ alkyl-group, more preferably a C₁-C₄ alkyl-group. More preferably the alcohol is selected from the group consisting of methanol, ethanol, n-propanol, i-propanol, n-butanol, s-butanol, t-butanol, The alkyl-group Rx may be optionally substituted and optionally containing hetero-atoms such as S, O, N. Preferably the acid hydrolysis is performed in methanol or ethanol. Step V shows the acid or basic hydrolysis of the dinitrile into a diacid derivative, (f), of the corresponding isohexide. Step VI shows the acid or basic hydrolysis of the diester, (e), Into a diacid derivative, (f), of the corresponding isohexide. Step VII represents an acid catalyzed esterification of the diacid, (f), derivative of the corresponding isohexide. The diester, (e), obtained In step IV or VII may be reduced with e.g. LiAlH₄, In order to obtain the corresponding diol, (g) (diol 1), see step VIII. Step IX represents the preparation of the bisoxazoline derivative, (h), of an isohexide by reacting the corresponding dinitrile derivative with mono ethanolamine. Bisoxazolines may be prepared according to methods known in the art (see for example "synthesis of bisoxazolines and their application as chain extender for poly(ethylene terephtaiate)", T. Loontjens et al., Makromoleculare Chemie, Macromolecular symposia 1993, 75, 211-216). Finally, step X represents the preparation of a diisocyanate, (i), by reacting the diamine obtained in step III with phosgene, diphosgene, triphosgene, or when phosgene free chemistry is desired with a carbonate or urea. Diisocyanate derivatives may be prepared according to methods known in the art (see for example the polyurethanes book, Huntsman international LLC, ed. D. Randall and S. Lee, Wiley, 2002).

Because of the nature of the chemistry employed here, i.e. second order nucleophilic substitution (S_{N}2) reactions, the stereo- and regio-chemistry of the products is governed by the stereo- and regio-chemistry of the starting materials. When isomannide with 2 endo-hydroxyl groups at positions 2 and 5 (position numbers, see Formula 1) is used as starting material, the product will have the new substituents In the exo-exo configuration (i.e. isoidide configuration).

In an embodiment mono-substituted compounds derived from corresponding isohexides may be prepared by a process which is schematically shown in Figure 3 and Figure 4. In a first step (i.e. step XI in Figure 3) an isohexide, (a), is provided with one protective group (PG). For this purpose, standard protective groups for alcohols may be used, e.g. benzylethers, silylethers, esters. After step XI a protected isohexide, (j), may be obtained. In a second step (step XII), the protected isohexide, (j), may be reacted with a triflate reagent, e.g. (CF₃SO₂)₂O or CF₃SO₂Cl, in order to provide a leaving group on the 2 or 5 position of the bicyclic isohexide. This step is comparable to step I as previously described, but may yield a monoactivated isohexide, (k), i.e. an isohexide provided with one leaving group (LG) and one protective group (PG), see Figure 3. In a subsequent step, step XIII, the monoactivated isohexide, (k), may be reacted with e.g. NaCN in THF to obtain a protected mononitrile, (I). Finally the protective group may be removed by methods known In the art (step XIV), in order to obtain a mononitrile derivative, (m), of the corresponding isohexide. Upon removal of the protective group the conformation of the original isohexlde OH-group is maintained.

Also In this embodiment the obtained mononitrile may be used as a platform chemical for preparing numerous derivatives of which 6 alternatives are shown In Figure 4. The involved reactions are similar to the reactions described earlier for steps III to X of Figure 2 and comprise: reacting the mononitrile, (m), with e.g. LiAlH₄ or H₂ and a catalyst (step XV), i.e. a reduction of the mononitrile into an aminoalcohol, (n). Step XVI shows the acid hydrolysis of the mononitrile, (m), into a hydroxyester (monoester), (o), by reacting the mononitrile with an alcohol with general formula Rx-OH, wherein Rx may be a branched or unbranched C₁-C₂₂ alkyl-group, preferably a C₁-C₁₀ alkyl-group, more preferably a C₁-C₄ alkyl-group. More preferably the alcohol is selected from the group consisting of methanol, ethanol, n-propanol, i-propanal, n-butanol, s-butanol, t-butenol. The alkyl-group Rx may be optionally substituted and optionally containing hetero-atoms such as S, O, N. Preferably the acid hydrolysis is performed in methanol or ethanol. Step XVII shows the acid or basic hydrolysis of the mononitrile, (m), Into a hydroxyacid derivative, (p), of the corresponding isohexide. Step XVIII shows the acid or basic hydrolysis of the hydroxyester, (o), Into a hydroxyacid derivative, (p), of the corresponding isohexide. Step XIX represents an acid catalyzed (mono)esterification of the hydroxyacid, (p), derivative of the corresponding isohexide. The hydroxyester obtained In step XVI or XIX may be reduced with e.g. LiAlH₄, in order to obtain the corresponding diol, (q), (i.e. diol 2, having one -OH directly connected to the bicyclic isohexide), see step XX. Step XXI represents the preparation of the hydroxyloxazoline derivative, (r), of an isohexide by reacting the corresponding mononitrile derivative with mono ethanolamine. Finally, step XXII represents the preparation of an isocyanate, (s), by reacting the aminoalcohol obtained in step XV with phosgene, diphosgene or triphosgene.

The processes according to the present Invention are known to be the first successful 1∼carbon extension on the 2- and 5-position on an isohexide ring system, starting from the easily obtainable parent isohexides.

In other words, the present invention also relates to a process for preparing a compound according to Formula 2

wherein the R¹ and R² may be the same or different and selected from -OH and a reactive group, the reactive group comprising a bridging carbon atom being directly bonded to the bicyclic isohexlde, provided that R¹ and R² are not both -OH. Preferably R¹ and R² are Independently from one another selected from the group consisting of -OH;-CN; -COOH; -CH₂NH₂; -CH₂OH; -(C=O)-O-Rx; -CH₂NCO, and oxazoline, provided that R¹ and R² are not both -OH and wherein Rx may be a branched or unbranched C₁-C₂₂ alkyl-group, preferably a C₁-C₁₀ alkyl-group, more preferably a C₁-C₄ alkyl-group, more preferably an alkyl-group selected from the group consisting of methyl, ethyl, n-propyl, i-propyl, n-butyl, s-butyl, t-butyl. Most preferably Rx is methyl or ethyl, The alkyl-group Rx may be optionally substituted and optionally containing hetero-atoms such as S, O, N. The process comprises the sequential steps of:
1) providing an isohexide selected from the group consisting of Isosorbide, isomannide and isoldide as starting material and having the general formula 1
2) introducing at least one leaving group on the 2 and/or 5 position of the bicyclic isohexide in order to obtain an at least partly activated isohexide;
3) substituting the at least one leaving groups by a -CN group; and
4) optionally converting the -CN group into a functional group.

With the above process, a product with a general formula according to formula 2, with at least one -CN group as R¹ and/or R² -group may be obtained.

Optionally, the -CN group may for example be converted into a -COOH, -CH₂NH₂, -CH₂OH, -(C=O)-O-Rx, -CH₂NCO, or oxazoline group; wherein Rx may be a branched or unbranched C₁-C₂₂ alkyl-group, preferably a C₁-C₁₀ alkyl-group.

In an embodiment the process according to the invention comprises the additional step of providing a protective group (PG) on the 2 or 5 position of the bicyclic isohexide, wherein the protective group is introduced prior to step 2.

In an embodiment, step 4) of the process according to the invention comprises the step of:
1. hydrolyzing the product obtained in step 3).

With the process according to this embodiment of the present invention, a compound having a general formula according formula 2 and having at least one -COOH group as R¹ and/or R² -group may be obtained.

In an embodiment the hydrolysis may be a homogeneous or heterogeneous acidic or basic hydrolysis.

In an embodiment the hydrolyses comprises solvolysis which may be performed in an alcohol selected from the group containing methanol, ethanol, n-propanol, i- propanol, n-butanol, s-butanol, t-butanol, preferably methanol or ethanol is selected.

The solvolysis may be performed in the presence of HCl. The mono- or dinitrile may be fully converted, or at least converted to a large extent, into a mixture of monoester and hydroxyacid (i.e. a mixture of compounds according to formula 2 wherein one of the R¹ and R²-groups Is -OH and the other is -COOH or -(C=O)-O-Rx, wherein the Rx group comprises the previously indicated groups; see figure 4), or - in case of a dinitrile intermediate - a mixture of the mono- and dialkylester and the diacid of the corresponding isohexide (i.e. a mixture of compounds according to formula 2 with R¹ and/or R² being - (C=O)-O-Rx or-COOH; see figure 2). The hydroxyacid, the diacid and the monoalkylester may further react with the solvent (which is an alcohol) to be converted into the corresponding monoester or diester. Preferably methanol is used as a solvent, such that - Rx is -CH₃.

The mixture may be easily purified e.g. by distillation, extraction, chromatography or (re)crystallization.

In an embodiment, step 4) of the process according to the present invention comprises the steps of:
i. hydrolyzing the product obtained in step 3)
ii. esterifying the product obtained in step i with a C₁-C₂₂ alcohol; and
iii. optionally reducing the product obtained in step ii.

The hydroxyacid or dlacid obtained In step i may be first esterified with a C₁-C₂₂ alcohol, preferably methanol and a homogeneous acid catalyst, e.g. HCl or H₂SO₄ and then reduced to a diol of the general formula 2, wherein at least one of the R¹ and R² is - CH₂-OH.

In an embodiment, step 4) of the process according to the invention, comprises the step of:
a) reducing the product obtained in step 3).

The mono- or dinitrile obtained in step 3 may be reduced to an aminoalcohol or diamine respectively of the general formula 3, wherein at least one of the R¹ and R² is -CH₂-NH₂. The reduction step (step a) may be performed with e.g. LiAlH₄ or H₂ with a catalyst.

In an embodiment, step 4) of the process according to the present invention, comprises the steps of:
a) reducing the product obtained in step 3); and
b) reacting the product obtained in step a) with phosgene, diphosgene, triphosgene, carbonate or urea.

The aminoalcohol or diamine obtained in step a) may be reacted with phosgene, diphosgene, triphosgene or when phosgene free chemistry Is desired with a carbonate or urea to obtain the corresponding Isocyanate and diisocyanate respectively.

In an embodiment, step 4) of the process according to the present invention, comprises the step of:
- reacting the product obtained in step 3) with mono ethanolamine.

The mono- or dinitrile obtained in step 3, may be converted Into the hydroxyoxazoline or bisoxazoline derivative of the corresponding isohexide.

In an embodiment, the process according to any other embodiment of the present invention, the at least one leaving groups Introduced in step 2 may be a triflate group, i.e. a trifluoromethane sulfonate group (-SO₂CF₃) which may originate from a triflate reagent, e.g. (CF₃SO₂)₂O or CF₃SO₂Cl.

In an embodiment, the process according to any other embodiment of the present invention comprises at least one Intermediate purification step.

The synthetic method described here is the most convenient direct way to a novel family of bio-based building blocks. Furthermore, this method ensures complete control over the stereo- and region-chemistry.

### EXPERIMENTAL METHODS AND MEASUREMENTS

### Nuclear Magnetic Resonance (NMR)

NMR spectra were recorded on a Bruker Avance III spectrometer operating at 400.17 MHz (¹H) and 100.62 MHz (¹³C) at room temperature.

### Gas chromatography (GC)

Gas chromatography was performed on an interscience Focus GC equipped with an AS 3000 series auto sampler. Injection volume 1 µl. Injector temperature 275°C. Split ratio 1:33. Column flow (at 275°C) 50 ml/min Helium. GC column: Restek Rxi-5ms. 30m x 0.25mm x 0.25µm. GC program (2,5-FDA.mth); Hold 2 min at 70°C, ramp 10°C/min, final temperature 300°C, hold 2 min. Total run time 27 min. Detector; FID at 300°C.

### Fourier transform infrared spectroscopy (FT-IR)

Fourier transform Infrared (FT-IR) spectra were obtained on a Varian Scimitar 1000 FT-IR spectrometer equipped with a Pike MIRacle ATR Diamond/ZnSe single reflection plate and a DTSG-detector. The measurement resolution was set at 4 cm⁻¹, and the spectra were collected In the range 4000-650 cm⁻¹ with 32 co-added scans.

### Methylation method; typical procedure for acid products

A sample of 5mg of material was weighed into a 2 ml GC vial and dissolved in 1ml of a 1:1 mixture of chloroform (Merck, p.a.) and methanol (Merck, p.a.). To 200uL of this solution was added 100uL of a 0.25M trimethylsulphonium hydroxide solution In MeOH (Fluka). These vials were manually shaken and directly used for GC analysis without further treatment.

### EXAMPLES

### Materials:

Isomannide (Sigma-Aldrich), Trifluoromethanesulfonic anhydride (>99%, Sigma-Aldrich), Dichloromethane (Merck, p.a.), Pyridine (Merck, p.a.), Hydrochloric acid (reagent grade 37%, Sigma-Aldrich), Tetrahydrofuran (anhydrous, Sigma-Aldrich), Potassium cyanide (extra pure, Merck), 18-Crown-6 (>99%, Fluka), Chloroform (Merck, p.a.), Methanol (Merck, p.a.), Lithium aluminum hydride (LIAIH₄ reagent grade, Sigma-Aldrich), Borane-Tatrahydrofuran complex (BH₃-THF) In THF solution (1.0M, Sigma-Aldrich), Hydrochloride In diethyl ether solution (2.0M, Sigma-Aldrich), Chloroform-D (99.8 atom% D Sigma-Aldrich h), D₂O (>99-8%, Merck), Methanol-d₄ (99.8 atom % D, contains 0.05 % (vlv) TMS, Sigma-Aldrich), Activated carbon (Norit, CN1), Magnesium sulfate (Acros Organics, 99% extra pure, dried, contains 3 to 4 moles of water), Celite® 545 coarse (Fluka). Amberlyst® A26 (Sigma-Aldrich) hydroxide form (Strongly basic, macroreticular resin with quaternary ammonium functionality from Rohm and Haas Co): prior to use, the resin was washed with demineralized water (100 ml) by sonication In an ultrasonic bath at room temperature for 10 min. The water layer was subsequently removed by decantation. This procedure was repeated 5 times, until the water layer remained colorless. All the chemicals were used as received, unless denoted otherwise.

### Experiment 1: Synthesis of Isomannidebistriflate (Figure 1: product b. reaction step 1):

A 500 mL three-necked round-bottom flask, equipped with mechanical stirrer and dropping funnel, was charged with Isomannide (36.5 g, 0.25 mol), pyridine (49 mL) and dichloromethane (150mL). The colorless solution was cooled to -10°C. Next, trifluoromethanesulfonic anhydride (0.6 mol, 100 mL) was added drop wise over 1 h. After stirring at room temperature for an additional 3 h, the reaction mixture was poured onto Ice-water (0.5 L) and stirred. The organic layer was separated, and the water layer was extracted with chloroform (3x150 mL). The combined organic layers were subsequently washed with aqueous HCI (1.0 M, 3x150 mL), water (2x150 mL), dried over MgSO₄, and decolorized with activated carbon. After filtration over a glass filter containing Cellte, the resulting clear solution was evaporated under reduced pressure using a rotary evaporator. Finally, pure isomannidebistriflate was obtained by recrystallization from ethanol as colorless needles.

Yield: 95 g, 93 % (purity: 99.9%, GC). ¹H NMR [(CDCI₃). δ, ppm]: 5.22 (dd, 2H), 4.77 (m, 2H), 4.15 (m, 4H). ¹³C NMR [(CDCI₃), δ, ppm]: 118.48 (q, ¹J_{FC}= 319 Hz), 83.43, 80.32, 70.86.

### Example 1: synthesis of the dlnitrile from isomannidebistriflate (Figure 1: product c. reaction step II):

A 500 mL, three-necked round-bottom flask, equipped with a magnetic stirrer, Internal thermo couple, pressure equalizing dropping funnel and a reflux condenser, was charged with potassium cyanide (28.5g, 440 mmol), 18-Crown-6 (28.91 g, 110 mmol) and THF (100 mL). The suspension was cooled down to -10°C, and then a solution of Isomannidebistriflate, e.g. as obtained from experiment 1 (30 g, 73 mmol) in THF (50 mL) was added drop wise over 1h h under stirring and continuous flow of nitrogen. During the course of the reaction, the reaction mixture first became yellow and then orange, while the internal temperature increased rapidly. After addition was complete, the reaction was stirred at 40°C for 24h under nitrogen. The color of reaction mixture gradually became darker, finally resulting in the formation of a black suspension. After cooling down to room temperature, the suspension was poured into cold water (200 mL) and extracted with chloroform (5x100 mL). The combined organic layers were dried over MgSO₄ and decolorized with activated carbon. After filtration over a glass filter containing Celite, the resulting solution was evaporated under reduced pressure using a rotary evaporator to give the crude product as a brown-black solid. Finally, pure dinitrile was obtained as a colorless solid by flash column chromatography (ethyl acetate/petroleum ether = 1:2).

Yield: 8.2 g, 68 % (purity: 98.7%, GC). IR (cm⁻¹): 2492, 2901, 2248 (CΞN), 1487, 1117, 1080, 1025, 947, 755. ¹H NMR [(CDCI₃), δ, ppm]: 5.02 (s, 2H), 4,14 (s, 4H), 3.19 (m, 2H). ¹³C NMR [(CDCI₃), δ, ppm]: 117.22, 86.20,70.34,36.70.

### Example 2: synthesis of the diamine from the dinitrile derivative of isoidide (Figure

### 2: product d, reaction step III):

### A) Synthesis of dlamine HCI salt:

A 250 mL three-necked round-bottom flask, equipped with a magnetic stirrer, pressure equalizing dropping funnel, and a reflux condenser, was charged with BH₃-THF complex in THF solution (1.0M, 50 mL). Next, a solution of dinitrile, compound c of Figures 1 and 2 (0.8g, 4.88 mmol), in THF (20 mL) was added drop wise at room temperature under a nitrogen flow in 20 min. After addition was complete, the reaction was stirred for another 16h. Next, methanol (30 mL) was added carefully to quench the reaction, during which hydrogen gas was released vigorously. When no more gas evolved, hydrogenchloride in diethyl ether solution (2.0M, 30 mL) was added drop wise and a white precipitate formed immediately. The suspension was then filtered over a glass filter (G-3), and the collected gum-like solid was dried In an oven (70°C, 1 atm.) for 1h, to give the crude diamine HCI salt as white hard powdered solid. Finally, the pure colorless diamine HCI salt was obtained after repeated washing with ethanol (3x 20 mL) and drying under vacuum.

Yield: 1.19 g, 66%. ¹H NMR [(D₂O), δ, ppm]: 4.45 (s, 2H), 4.01 (m, 2H), 3.67 (m, 2H), 3.54 (d, 4H), 2.42 (m, 2H). ¹³C NMR [(D₂O), δ, ppm]: 85.24, 89.79, 61.70, 49.38.

### B) Synthesis of diamine:

Diamine HCI salt, obtained as described above, (0.19g, 0.78 mmol) was dissolved in demineralized water (20 mL) giving a colorless solution. To this solution freshly washed Amberlyst A 26-OH (0.75g, 3.2mmol) was added. The resulting suspension was sonicated in an ultrasonic bath for 1 h at 30°C. After the reaction was complete, the suspension was filtered over a glass filter (G-3) containing Celite, and the IEX-resin was washed thoroughly with water (3x15 mL) and methanol (3x 15mL). The combined clear colorless solutions were evaporated to dryness using a rotary film evaporator to afford the pure diamine as a colorless solid.

Yield: 0.1 g, 74.6 % (purity 96.0%, ¹H NMR). ¹H NMR [(CD₃OD), δ, ppm]: 4.37 (s, 2H), 3.97 (m, 2H), 3.60 (m, 2H), 2.74 (m, 4H), 2.32(m, 2H). ¹³C NMR [(CD₃OD), δ, ppm]: 87.28, 71.51, 49.94, 42.63.

### Example 3: synthesis of the dimethyl ester from the dinitrile derivative of Isoldide (Figure 2: product e, reaction step IV):

A 100 mL one-necked round-bottom flask was equipped with a magnetic stirrer and a reflux condenser. Then methanol (20 mL), HCI (37%, 20 mL) and dinitrile, see compound c in Figures 1 and 2, (1.5g) were sequentially charged to the flask under stirring. After stirring at reflux for 3h, the reaction mixture was cooled to room temperature and extracted with chloroform (3x50 mL). The combined organic layers were dried over MgSO₄, and filtered over a glass filter containing Celite. The resulting solution was evaporated at reduced pressure using a rotary evaporator to give the crude product as a colorless oil. Finally, pure diester was obtained as a colorless liquid by flash column chromatography (ethyl acetate/petroleum ether = 1: 5).

Yield: 1.37g, 65% (purity 98.0%, ¹H NMR). IR (cm⁻¹): 1730 (C=O). ¹H NMR [(CDCI₃). 5, ppm]: 4.92 (s, 2H), 4.10 (m, 2H), 4.02 (m, 2H), 3.73 (s, 6H), 3.20 (t, 2H). ¹³C NMR [(CDCI₃), δ, ppm]: 171.51, 85.73, 69.82, 52.04, 51.32.

### Example 4: synthesis of the diacid from the diester derivative of isoidide (Figure 2: product f. reaction step VI):

A 100 mL one-necked round-bottom flask, equipped with a magnetic stirrer and a reflux condenser, was charged with diester, see compound e in Figure 2, (187 mg, 0.8 mmol) and aqueous HCI solution (1.0 M, 20 mL). The reaction mixture was stirred under reflux for 24 h and then cooled down to room temperature. After removal of the solvent under reduced pressure using a rotary evaporator, the crude diacid was obtained as a colorless oil, which was subsequently dissolved in refluxing diethyl ether (20 mL). Storing this solution overnight at -20°C resulted in the formation of a colorless solid. Decantation of the supernate, followed by repeated washing with cold diethyl ether (3x 20 mL), and drying under vacuum, gave the pure diacid as colorless solid.

Yield: 103 mg, 62.8 % (purity 96.0%, ¹H NMR). IR (cm⁻¹): 1692 (C=O). ¹H NMR [(D₂O), δ, ppm]: 4.98 (s, 2H), 4.12 (bm, 4H), 3.25 (bs, 2H). ¹³C NMR [(CDCI₃). δ, ppm]: 177.73, 83.30, 72.25, 53.86.

### Example 5: synthesis of the diol from the diester derivative of Isoidide (Figure 2: product g, reaction step VIII):

Dry THF (10 mL) and LiAIH₄ (0.22g, 6mmol) were charged into a 100 mL three-necked round-bottom flask equipped with a magnetic stirrer, pressure equalizing dropping funnel, and a reflux condenser. To this grey slurry a solution of diester, see e in Figure 2, (0.345g, 1.5 mmol) in dry THF (10 mL) was added, at 0°C, under a nitrogen atmosphere. When the addition was complete, the reaction was stirred at room temperature for 1 h and then refluxed for 4h. Next, the reaction mixture was cooled to 0°C, and carefully quenched with aqueous sodium hydroxide (10%, 0.3 mL), water (0.85 mL), and aqueous sodium hydroxide (10%, 0.2 mL). After stirring for 20 min., the mixture was diluted with ethyl acetate (EA, 30 mL), filtered through Celite (EA wash), and concentrated under reduced pressure to give 0.275g oil-like crude product. The pure diol was obtained as a colorless oil by flash chromatography (methanol/dichloromethane = 1:10).

Yield: 0.15 g, 58 % (purity 96.0%, ¹H NMR). ¹H NMR [(CDCI₃), δ, ppm]: 4.47 (s, 2H), 3.99 (m, 2H), 3.68 (m, 2H), 3.60 (d, 4H), 2.80 (bs, 1H, OH), 2.45 (m, 2H). ¹³C NMR ((CDCI₃), δ, ppm]: 85.48, 69.96, 62.16, 49.19.

## Claims

1. A compound which has the general formula: 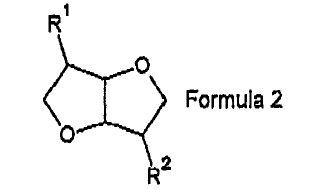 wherein the R¹ and R² may be the same or different and selected from -OH and a reactive group, the reactive group comprising a bridging carbon atom being directly bonded to the bicyclic isohexide, provided that R¹ and R² are not both -OH.

2. The compound according to claim 1, wherein R¹ and R² are Independently from one another selected from the group consisting of -OH: -CN; -COOH; -CH₂NH₂; - CH₂OH; -(C=O)-O-Rx; -CH₂NCO, and oxazoline, provided that R¹ and R² are not both -OH and wherein Rx may be a branched or unbranched C₁-C₂₂ alkyl-group.

3. The compound according to any one of claims 1 and 2, wherein the R¹ and R² are the same.

4. The compound according to any one of claims 1 and 2, wherein one of the R¹ and R² -groups comprises -OH and the other is selected from the group consisting of - CN, -COOH, -CH₂NH₂, -CH₂OH, -(C=O)-O-Rx, -CH₂NCO, and oxazoline; wherein Rx may be a branched or unbranched C₁-C₂₂ alkyl-group, preferably a C₁-C₁₀ alkyl-group.

5. A process for preparing a compound according to any one of claims 1-4, the process comprising the sequential steps of:
1) providing an Isohexide selected from the group consisting of isosorbide, isomannlde and isoidide as starting material and having the general formula: 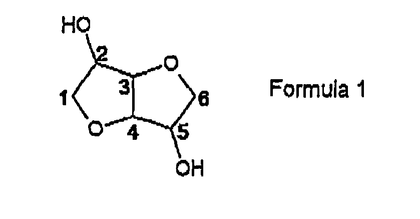
2) Introducing at least one leaving group on the 2 and/or 5 position of the bicyclic isohexide In order to obtain an at least partly activated isohexide;
3) substituting the at least one leaving groups by a -CN group; and
4) optionally converting the -CN group Into a functional group.

6. The process according to claim 5, comprising the additional step of providing a protective group (PG) on the 2 or 5 position of the bicyclic isohexide, wherein the protective group is introduced prior to step 2.

7. The process according to any one of claims 5 and 6, wherein step 4) comprises the step of:
i. hydrolyzing the product obtained In step 3).

8. The process according to claim 7, wherein step i comprises the solvolysis which is performed in an alcohol selected from the group consisting of methanol, ethanol, n-propanol, i- propanol, n-butanol, s-butanol, t-butanol, and optionally the solvolysis is performed In the presence of HCI.

9. The process according to any one of claims 5 and 6, wherein step 4) comprises the steps of:
i. hydrolyzing the product obtained In step 3);
ii. esterifying the product obtained in step i with a C₁-C₂₂ alcohol; and
iii. optionally reducing the product obtained in step ii.

10. The process according to claim 9, wherein the esterification step ii) is carried out In methanol and In the presence of a homogeneous acid catalyst selected from the group consisting of HCI and H₂SO₄.

11. The process according to any one of claims 5 and 6, wherein step 4) comprises the step of:
a. reducing the product obtained In step 3).

12. The process according to any one of claim 5 and 6, wherein step 4) comprises the steps of:
a. reducing the product obtained In step 3); and
b. reacting the product obtained In step a) with phosgene, diphosgene, triphosgene, carbonate or urea.

13. The process according to any one of claims 5 and 6, wherein step 4) comprises the step of:
- reacting the product obtained in step 3 with mono ethanolamine.

14. The process according to any one of claims 5-13, wherein in step 2 the isohexide obtained in step 1 Is reacted with a triflate reagent, optionally selected from the group consisting of (CF₃SO₂)₂O and CF₃SO₂CI In order to introduce at least one trifluoromethane sulfonate group as a leaving group on the 2 and/or 5 position of the bicyclic isohexide, and wherein the process optionally comprises at least one intermediate purification step.

15. Use of compounds according to any one of claims 1-4 or obtained by a process according to any one of claims 5-14, as blobased chiral building blocks in polymer technology, manufacturing of pharmaceuticals, liquid crystal technology and as ligand systems in catalysis.
